# EUROPEAN PATENT APPLICATION

(11) **EP 2 305 149 A1**
(43) Date of publication of application: **06.04.2011**
(21) Application number: 10251692.9
(22) Date of filing: 30.09.2010
(51) Int. Cl.: A61B 17/34

(54) **Port fixation with varying thread pitch**

(30) Priority: 01.10.2009 US 247661 P; 22.09.2010 US 887917
(71) Applicant: Tyco Healthcare Group LP, New Haven, CT 06511 (US)
(72) Inventor: Fischvogt, Gregory, Hamden, CT 06514 (US); Carter, Sally, Wallingford, CT 06492 (US); Fowler, David, Cheshire, CT 06410 (US)
(74) Representative: Soames, Candida Jane

(57) **Abstract**

A surgical portal device comprising a body portion and at least one thread is disclosed. The body portion defines a longitudinal axis and has a proximal end, a distal end, an exterior surface, and a lumen configured to allow a surgical instrument to pass therethrough. The at least one thread extends at least partially along the exterior surface of the body portion. The at least one thread defines a thread pitch between adjacent portions of the thread. A distal portion of the thread pitch is different from a proximal portion of the thread pitch.

## Description

### CROSS REFERENCE TO RELATED APPLICATION

The present application claims the benefit of and priority to U.S. Provisional Application Serial No. 61/247,661 filed on October 1,2009, the entire contents of which are incorporated herein by reference.

### BACKGROUND

### Technical Field

The present disclosure relates to surgical ports. More particularly, the present disclosure relates to surgical access ports having port fixation components to secure the surgical access port relative to tissue of a patient.

### Background of Related Art

Surgical ports, such as introducers, trocars, and cannulas, permit the introduction of a variety of surgical instruments into a body cavity or opening within a patient. In procedures, such as endoscopic, laparoscopic or arthroscopic surgeries, a passage is created through tissue to access an underlying surgical site in the body. A port or cannula is positioned within the passage. Surgical instruments are introduced within the cannula to perform a surgical procedure.

It may be advantageous to provide a portal device that can be removably placed within an incision or body opening of a patient to fix the access device therein.

### SUMMARY

The present disclosure relates to a surgical portal device comprising a body portion and at least one thread. The body portion defines a longitudinal axis and has a proximal end, a distal end, an exterior surface, and a lumen configured to allow a surgical instrument to pass therethrough. The at least one thread extends at least partially along the exterior surface of the body portion. The at least one thread defines a thread pitch between adjacent portions of the thread. A distal portion of the thread pitch is different from a proximal portion of the thread pitch.

The present disclosure also relates to a surgical portal device comprising a body portion and a threaded portion. The body portion defines a longitudinal axis and has a proximal end, a distal end, an exterior surface, and a lumen configured to allow a surgical instrument to pass therethrough. The threaded portion includes a first thread and a second thread. The first thread is interwoven with respect to the second thread along the entire length of the first thread.

### BRIEF DESCRIPTION OF THE DRAWINGS

Embodiments of the presently disclosed surgical portal device are described herein with reference to the accompanying drawings, wherein:

FIG. 1 is a perspective view of a surgical portal device according to an embodiment of the present disclosure;

FIG. 2 is a longitudinal cross-sectional view of the surgical portal device of FIG.1 illustrated partially within tissue;

FIG. 3 is a perspective view of a surgical portal device according to another embodiment of the present disclosure;

FIG. 4 is a longitudinal cross-sectional view of the surgical portal device of FIG. 3 illustrated partially within tissue; and

FIG. 5 is a flow chart illustrating a surgical method incorporating the surgical portal device.

Other features of the present disclosure will become apparent from the following detailed description, taken in conjunction with the accompanying drawings, which illustrate, by way of example, various principles of the present disclosure.

### DETAILED DESCRIPTION OF THE EMBODIMENTS

Particular embodiments of the present disclosure will be described herein with reference to the accompanying drawings. As shown in the drawings and as described throughout the following description, and as is traditional when referring to relative positioning on an object, the term "proximal" refers to the portion of the apparatus that is closer to the user and the term "distal" refers to the portion of the apparatus that is farther from the user. In the following description, well-known functions or constructions are not described in detail to avoid obscuring the present disclosure in unnecessary detail.

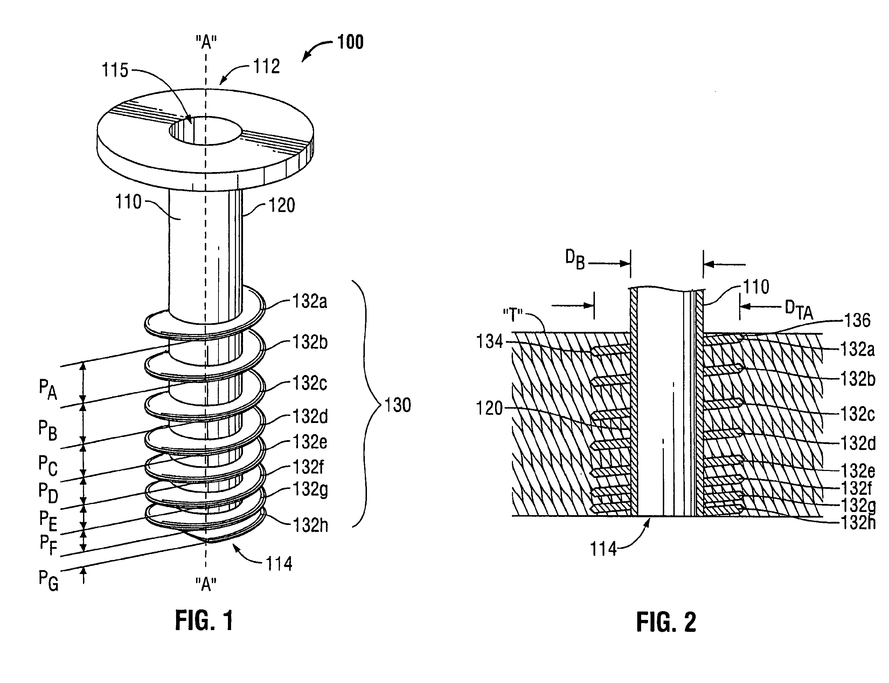

With reference to FIG. 1, a surgical portal device 100 in accordance with embodiments of the present disclosure is shown. The surgical portal device 100 includes a body portion 110 and a threaded portion 130. The body portion 110 includes a proximal end 112, a distal end 114, and a cylindrical bore or lumen 115 extending therethrough and defining a longitudinal axis "A-A." The lumen 115 is dimensioned for reception of at least one surgical instrument (not shown), including, but not limited to, clip appliers, graspers, dissectors, retractors, staplers, laser probes, photographic devices, endoscopes and laparoscopes, tubes, and the like.

The threaded portion 130 includes a plurality of thread segments 132a, 132b, etc. (collectively referred to as "thread segment 132" or "thread segments 132"). As used herein, the term "thread segment" relates to a single revolution of the threaded portion 130. While the term "thread segments" is used to describe portions of the threaded portion 130, "thread segments" is not intended to mean or suggest that the threaded portion 130 is not a single continuous thread. Rather, the threaded portion 130 may include a single continuous thread or a plurality of discontinuous threads, as is discussed in more detail below.

The body portion 110 includes an outer surface 120 having a substantially constant outer diameter D_{B} along a majority of its length. Each thread segment 132 includes a thread diameter (e.g., D_{TA} is the thread diameter of thread segment 132a) defined as the distance between opposite lateral outer edges 134, 136 of the thread segment 132 (see FIG. 2). Additionally, a thread pitch "P" is defined as the distance between adjacent tread segments 132. In the embodiment illustrated in FIGS. 1 and 2, the proximal-most thread segment is labeled 132a and the distal-most thread segment is labeled 132k. While eight thread segments (i.e., 132a, 132b, 132c, 132d, 132e, 132f, 132g and 132h) are illustrated, it is envisioned that body portion 110 includes more or fewer thread segments 132 therearound.

In the embodiment illustrated in FIGS. 1 and 2, the thread pitch gradually decreases from the distance between proximal-most thread segments 132a and 132b to the distance between distal-most thread segments 132g and 132h. That is, the proximal-most thread pitch P_{A} is larger than distally adjacent thread pitch P_{B}, which is larger than distally adjacent thread pitch P_{C}, etc. This gradual decrease in thread pitch continues towards the distal end 114 of body portion, where the distal-most thread pitch P_{G} is smaller than proximally adjacent thread pitch P_{F}. It is envisioned that the thread pitch P_{A} between proximal-most thread segments 132a and 132b is between about 0.15 inches and about 0.25 inches. It is envisioned that the thread pitch P_{G} between the distal-most thread segments 132g and 132h is between about 0.01 inches and about 0.10 inches. However, any pitch range is conceivable.

Additionally, while the embodiment illustrated in FIGS. 1 and 2 depicts a substantially linear change in thread pitch (i.e., the difference between adjacent thread pitches is substantially constant between the proximal-most thread pitch P_{A} and the distal-most thread pitch P_{G}), it is envisioned that the change in thread pitch along body portion 110 is non-linear (i.e., the difference between adjacent thread pitches is any combination of increasing, decreasing, and remaining constant between the proximal-most thread pitch P_{A} and the distal-most thread pitch P_{G}).

It is also envisioned that the threaded portion 130 includes a proximal portion comprising the proximal-most four thread segments, for example, and a distal portion comprising the distal-most four thread segments, for example. In such an embodiment, it is envisioned that the proximal portion includes a first, relatively large and substantially constant thread pitch between adjacent thread sections, and that the distal portion includes a second, relatively small and substantially constant thread pitch between adjacent thread sections.

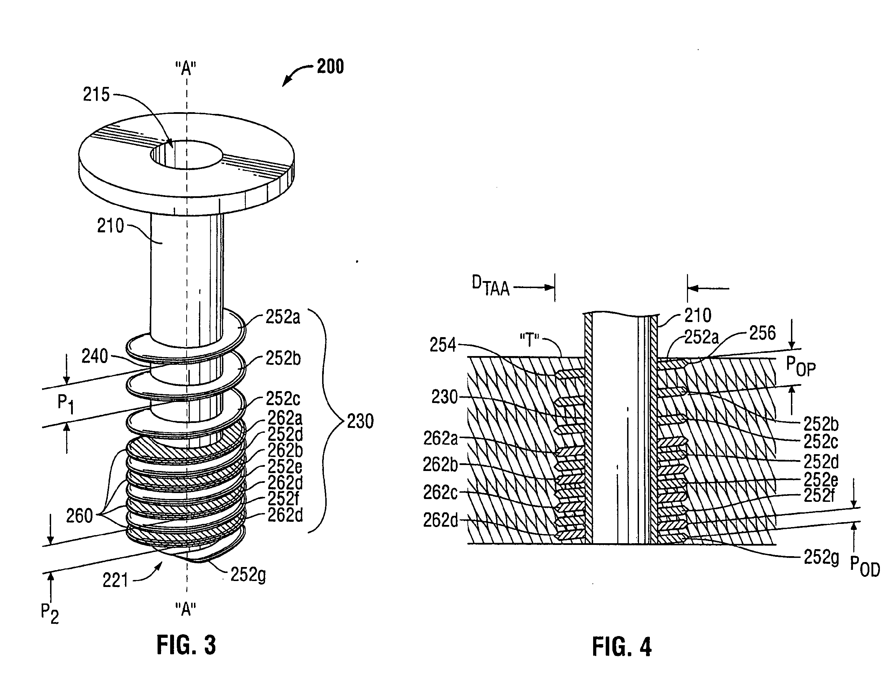

With reference to FIGS. 3 and 4, another embodiment of surgical portal device 200 is shown. Surgical portal device 200 of this embodiment includes threaded portion 230. In contrast to the embodiment of FIGS. 1 and 2, threaded portion 230 includes a first thread 250 and a second thread 260. Similarly to the embodiment of FIGS. 1 and 2, each thread 250, 260 includes a plurality of thread segments 252a, 252b, etc. (collectively referred to as "thread segment 252"), and 262a, 262b, etc. (collectively referred to as "thread segment 262"), respectively. While the term "thread segments" is used to describe portions of the threads 250 and 260, "thread segments" is not intended to mean or suggest that each of threads 250 and 260 is not a single continuous thread. Rather, threads 250 and 260 may include a single continuous thread or a plurality of discontinuous threads.

Each thread segment 252, 262 includes a thread diameter (e.g., D_{TAA} is the thread diameter of thread segment 252a) defined as the distance between opposite lateral outer edges 254, 256 of the thread segment 252 (see FIG. 4). Additionally, a thread pitch "P" is defined as the distance between adjacent tread segments 252 or 262. In the embodiment illustrated in FIGS. 3 and 4, the proximal-most thread segment of thread 252 is labeled 252a and the distal-most thread segment of thread 250 is labeled 252g. While seven thread segments (i.e., 252a, 252b, 252c, 252d, 252e, 252f and 252g) are illustrated, it is envisioned that thread 250 includes more or fewer thread segments 252. Additionally, in the embodiment illustrated in FIGS. 3 and 4, the proximal-most thread segment of thread 260 is labeled 262a and the distal-most thread segment of thread 260 is labeled 262d. While four thread segments (i.e., 262a, 262b, 262c and 262d) are illustrated, it is envisioned that thread 260 includes more or fewer thread segments 262.

With continued reference to FIGS. 3 and 4, thread 250 is shown as extending along a majority of the length of body portion 210, while thread 260 is shown extending only along a distal portion of body portion 210. Additionally, each thread segment 260 is shown between adjacent thread segments 250. That is, thread 260 is interwoven with thread 250. For viewing clarity and to facilitate distinguishing threads 250 and 260 from each other, thread 260 is shown having hatched marks in FIG. 3 and different hatched marks from thread 250 in FIG. 4.

In the illustrated embodiment, each thread 250, 260 has a substantially constant thread pitch, however it is within the scope of the present disclosure that thread 250 and/or thread 260 may include any combination of an increasing, decreasing, or constant thread pitch. In FIGS. 3 and 4, thread 250 includes thread pitch P₁ and thread 260 includes thread pitch P₂. It is envisioned that each of thread pitch P₁ and thread pitch P₂ is between about 0.15 inches and about 0.25 inches. However, due to the interweaving of threads 250 and 260, the overall thread pitch P_{O} is non-constant along the length of body portion 210. The overall thread pitch P_{O} is defined herein as the distance between adjacent thread sections. That is, at the locations where there is a single thread 250, the overall thread pitch P_{O} is equal to thread pitch P₁; at the locations where both threads 250 and 260 are present, the overall thread pitch P_{O} is a distance between adjacent thread sections 252 and 262. More particularly, and as shown in FIGS. 3 and 4, the overall thread pitch P_{OD} of the distal portion of body portion 210 is smaller (i.e., approximately half) than the overall thread pitch P_{OP} of the proximal portion of body portion 210. Accordingly, it is envisioned that the overall thread pitch P_{OP} of the proximal portion is between about 0.15 inches and about 0.25 inches, and it is envisioned that the overall thread pitch P_{OD} of the distal portion is between about 0.075 inches and about 0.125 inches. However, any thread pitch range is conceivable.

With reference to FIGS. 1-4, the thread diameters and thread pitch in the illustrated embodiments are illustrative only and the sizes shown are for clarity. As can be appreciated, smaller or larger thread diameters (relative to the exterior circumference of body portion 110, 210) and/or thread pitches are envisioned and within the scope of the present disclosure. Additionally, while the diameters of respective thread segments of threaded portions 130, 250 and 260 are shown as being constant, it is envisioned that the diameter of a thread section is different from the diameter of an adjacent thread section.

In accordance with the embodiments of the present disclosure, thread segments 132, 252, 262 of surgical portal device 100, 200 help removably secure surgical portal device 100, 200 within tissue "T." As can be appreciated, it is envisioned that the relatively small distal thread segments facilitate entry of surgical portal device 100, 200 into an incision, as it may reduce the trauma to the surrounding tissue "T" (see FIGS. 2 and 4). It is also envisioned that the relatively large proximal thread segments increase the amount of fixation within tissue "T." This provides a greater resistance to a proximally-directed force, which helps maintain the relative longitudinal position of surgical portal device 100, 200 with respect to adjacent tissue "T,"

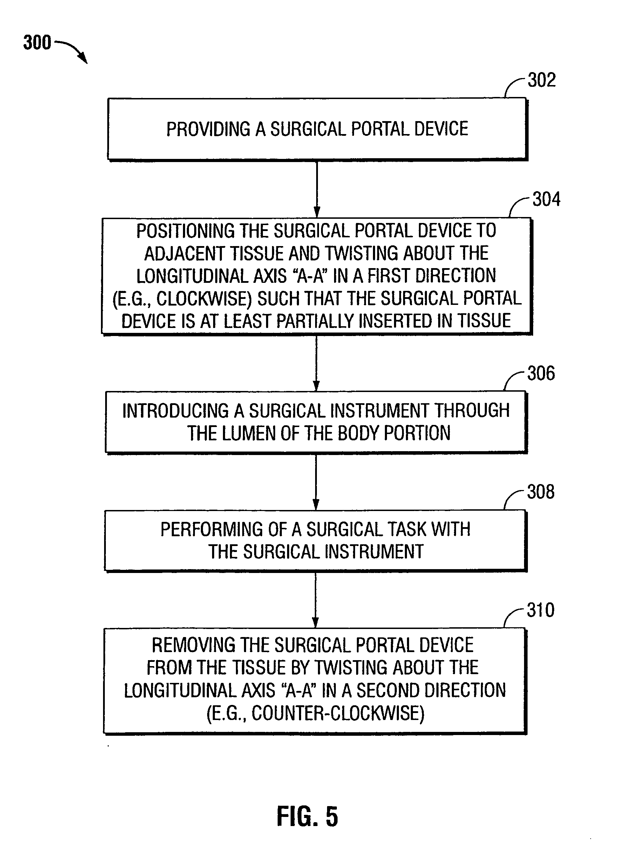

The present disclosure also relates to surgical methods utilizing the surgical portal device 100, 200. FIG. 5 is a flow chart illustrating a method 300 of use of the surgical portal device 100, 200. In accordance with the method 300, the surgical portal device 100, 200 including threaded portion 130, 250, 260 is provided (Step 302). The surgical portal device 100, 200 is positioned adjacent tissue and is twisted about the longitudinal axis "A-A" in a first direction (e.g., clockwise) such that the surgical portal device 100, 200 is at least partially inserted in tissue (Step 304). A surgical instrument is introduced through the lumen 115, 215 of the body portion 110, 210 (Step 306) followed by performance of a surgical task with the surgical instrument (Step 308). The surgical portal device 100, 200 may be removed from the tissue by being twisted about the longitudinal axis "A-A" in a second direction (e.g., counter-clockwise) (Step 310).

It will be understood that various modifications may be made to the embodiments of the presently disclosed portal device. Therefore, the above description should not be construed as limiting, but merely as exemplifications of embodiments. Those skilled in the art will envision other modifications within the scope and spirit of the present disclosure.

## Claims

1. A surgical portal device comprising:
a body portion defining a longitudinal axis and having a proximal end, a distal end, an exterior surface, and a lumen configured to allow a surgical instrument to pass therethrough; and
at least one thread extending at least partially along the exterior surface of the body portion, wherein the at least one thread defines a thread pitch between adjacent portions of the thread, a distal portion of the thread pitch being different from a proximal portion of the thread pitch.

2. The surgical portal device of claim 1, wherein the distal portion of the thread pitch is less than the proximal portion of the thread.

3. The surgical portal device of claim 1 or claim 2, wherein the at least one thread is a single continuous thread.

4. The surgical portal device of claim 1 or claim 2, wherein the at least one thread includes a plurality of discontinuous threads.

5. The surgical portal device of any preceding claim, wherein the exterior surface of the body portion has a substantially constant exterior circumference along a majority of its length.

6. The surgical portal device of any preceding claim, wherein the distal portion of the thread pitch is substantially constant and the proximal portion of the thread pitch is substantially constant.

7. The surgical portal device of any of claims 1 to 5, wherein the thread pitch gradually increases from the distal portion of the thread to the proximal portion of the thread.

8. A surgical portal device comprising:
a body portion defining a longitudinal axis and having a proximal end, a distal end, an exterior surface, and a lumen configured to allow a surgical instrument to pass therethrough; and
a threaded portion including a first thread and a second thread, the first thread being interwoven with respect to the second thread along the entire length of the first thread.

9. The surgical portal device of claim 8, wherein a thread pitch of the first thread is constant and wherein a thread pitch of the second thread is constant.

10. The surgical portal device of claim 9, wherein the thread pitch of the first thread is substantially equal to the thread pitch of the second thread.

11. The surgical portal device of any of claims 8 to 10, wherein each of the first thread and the second thread is continuous.

12. The surgical portal device of any of claims 8 to 11, wherein the second thread includes a longer length than the first thread.

13. The surgical portal device of any of claims 8 to 12, wherein the second thread extends proximally beyond a proximal-most portion of the first thread.

14. A surgical portal device comprising:
a body portion dimensioned for positioning within body tissue, the body portion defining a longitudinal axis and having a proximal end, a distal end, an exterior surface, and a lumen configured to allow a surgical instrument to pass therethrough; and
at least one thread extending at least partially along the exterior surface of the body portion, the at least one thread defining a thread pitch which varies along a longitudinal segment of the body portion.
